(19) Europäisches Patentamt
European Patent Office
Office européen des brevets

(11) Veröffentlichungsnummer : **0 395 837 B1**

(12) # EUROPÄISCHE PATENTSCHRIFT

(45) Veröffentlichungstag der Patentschrift :
**14.04.93 Patentblatt 93/15**

(51) Int. Cl.$^5$ : **A61K 7/13,** C07C 217/76

(21) Anmeldenummer : **90101961.2**

(22) Anmeldetag : **01.02.90**

(54) **Oxidationshaarfärbemittel auf der Basis von 4-Aminophenol-derivaten sowie neue 4-Aminophenol-derivate.**

(30) Priorität : **29.04.89 DE 3914253**

(43) Veröffentlichungstag der Anmeldung :
**07.11.90 Patentblatt 90/45**

(45) Bekanntmachung des Hinweises auf die Patenterteilung :
**14.04.93 Patentblatt 93/15**

(84) Benannte Vertragsstaaten :
**DE ES FR GB IT**

(56) Entgegenhaltungen :
**EP-A- 0 182 187**
**EP-A- 0 359 618**

(73) Patentinhaber : **Wella Aktiengesellschaft**
**Berliner Allee 65**
**W-6100 Darmstadt (DE)**

(72) Erfinder : **Clausen, Thomas, Dr.**
**Ernst-Pasqué-Strasse 35 A**
**W-6146 Alsbach (DE)**
Erfinder : **Balzer, Wolfgang, Dr.**
**Im Kiessling 12**
**W-6146 Alsbach (DE)**
Erfinder : **Flohr, Anke**
**Alte Falterstrasse 29**
**W-6230 Frankfurt 80 (DE)**

EP 0 395 837 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Oxidationshaarfärbemittel auf der Basis von 4-Aminophenol-derivaten sowie neue 4- Aminophenol-derivate

Gegenstand der Erfindung sind Mittel zur oxidativen Färbung von Haaren auf der Basis von bestimmten 4-Aminophenol-derivaten als Entwicklersubstanz sowie neue 4-Aminophenol-derivate.

In der Haarfärbepraxis haben die Oxidationsfarbstoffe eine wesentliche Bedeutung erlangt. Hierbei werden die Farbstoffe durch oxidative Kupplung von Entwicklersubstanzen und Kupplersubstanzen im Haarschaft erzeugt. Dies führt zu sehr intensiven Haarfärbungen mit sehr guter Farbechtheit. Außerdem können durch die Kombination geeigneter Entwickler- und Kupplersubstanzen unterschiedliche Farbnuancen erzeugt werden.

Als Entwicklersubstanzen werden insbesondere 2,5-Diaminotoluol, 2,5-Diaminobenzylalkohol, 2,5-Diaminophenylethylalkohol, p-Aminophenol und 1,4-Diaminobenzol eingesetzt. Von den vorzugsweise verwendeten Kupplersubstanzen sind Resorcin, 4-Chlorresorcin, 1-Naphthol, m-Aminophenol und Derivate des m-Phenylendiamins zu nennen.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert.

Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwickler- und Kupplerkomponenten eine breite Palette verschiedener Farbnuancen erzeugt werden kann.

Zur Erzielung natürlicher und besonders modischer Nuancen im Rotbereich wird vor allem p-Aminophenol, allein oder im Gemisch mit anderen Entwicklersubstanzen, in Kombination mit geeigneten Kupplersubstanzen eingesetzt.

Gegen die für den Rotbereich der Farbskala bisher hauptsächlich eingesetzte Entwicklersubstanz p-Aminophenol werden in letzter Zeit Bedenken in bezug auf die physiologische Verträglichkeit erhoben, während die in neuerer Zeit empfohlenen Entwicklersubstanzen, wie zum Beispiel Pyrimidinderivate, in färberischer Hinsicht nicht völlig zufriedenstellen können.

Es wurden bereits Versuche unternommen, den Nachteil der schlechten physiologischen Verträglichkeit des bisher für den Rotbereich eingesetzten Entwicklersubstanz p-Aminophenol zu beheben.

So sind aus den Veröffentlichungen DE-OS 3 441 148 und DE-OS 3 538 750 Oxidationshaarfärbemittel mit einem Gehalt an 4-Amino-2-hydroxymethylphenol bekannt, die bei einer gegenüber dem p-Aminophenol geringfügig verbesserten physiologischen Verträglichkeit nur zu zufriedenstellenden Haarfärbungen im Rotbereich führen.

Es bestand daher die Aufgabe, ein Oxidationshaarfärbemittel auf der Basis von Entwicklersubstanzen für den Rotbereich zur Verfügung zu stellen, die dem p-Aminophenol in Farbbrillianz und Farbintensität gleichkommen, jedoch eine deutlich bessere physiologische Verträglichkeit aufweisen.

Hierzu wurde nun gefunden, daß durch ein Mittel zur oxidativen Färbung von Haaren mit einem Gehalt an mindestens einer Entwicklersubstanz und mindestens einer Kupplersubstanz, welches als Entwicklersubstanz ein 4-Aminophenol-derivat der allgemeinen Formel (I),

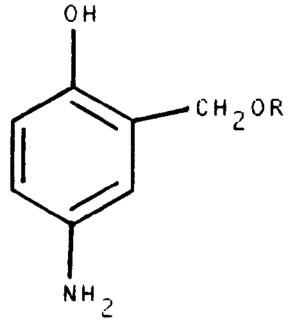

(I),

wobei R einen Propyl- oder iso-Propylrest, einen Aminoalkylrest mit 2 bis 4 Kohlenstoffatomen, einen Aminoalkylrest mit 2 bis 4 Kohlenstoffatomen, der an der Aminogruppe mit einem oder zwei Alkylresten, die 1 bis 4 Kohlenstoffatome aufweisen, substituiert ist, oder einen Dihydroxyalkylrest mit 3 bis 4 Kohlenstoffatomen bedeutet, oder dessen physiologisch vertragliches, wasserlösliches Salz enthält, die gestellte Aufgabe in hervorragender Weise gelöst wird. Die wasserlöslichen Salze der Verbindungen der allgemeinen Formel (I) sind durch Umsetzungen mit geeigneten organischen und anorganischen Säuren und Basen erhältlich.

Als physiologisch verträgliche, wasserlösliche Salze seien beispielsweise das Chlorid, das Sulfat, das Phophat, das Acetat, das Lactat und das Citrat genannt. Die Verbindungen der allgemeinen Formel (I) sind gut wasserlöslich und weisen insbesondere als Bestandteil des beschriebenen erfindungsgemäßen Haarfärbemittels eine ausgezeichnete Lagerstabilität auf.

In dem Haarfärbemittel sollen die erfindungsgemäßen Entwicklersubstanzen, von denen das 4-Amino-2-propoxymethyl-phenol und das 4-Amino-2-iso-propoxymethyl-phenol bevorzugt sind, in einer für die Haarfärbung ausreichenden Menge, im allgemeinen in einer Menge von etwa 0,01 bis 3,0 Gewichtsprozent, vorzugsweise 0,1 bis 2,5 Gewichtsprozent, enthalten sein.

Obwohl die vorteilhatten Eigenschaften der hier beschriebenen neuen Entwicklersubstanzen es nahelegen, diese als alleinige Entwickler zu verwenden, ist es selbstverständlisch auch möglich, die neuen Entwicklersubstanzen der Formel (I) gemeinsam mit bekannten Entwicklersubstanzen, wie zum Beispiel 1,4-Diaminobenzol, 2,5-Diaminotoluol oder 2,5-Diaminobenzylakohol, einzusetzen.

Von den bekannten Kupplersubstanzen kommen als Bestandtiel des hier beschriebenen Haarfärbemittels vor allem 1-Naphthol, Resorcin, 4-Chlorresorcin, 4,6-Dichlorresorcin, 2-Methylresorcin, 2-Amino-4-(2′-hydroxyethylamino)-anisol, 2,4-Diaminobenzylalkohol, m-Phenylendiamin, 5-Amino-2-methylphenol, 2,4-Diaminophenoxyethanol, m-Aminophenol, 3-Amino-4-chlor-6-methylphenol, 3-Amino-2-methylphenol, 4-Amino-2-hydroxy-phenoxyethanol, 4-Hydroxy-1,2-methylendioxybenzol, 4-(2′-Hydroxyethylamino)-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin in Betracht, wobei die einzelnen Kupplersubstanzen in dem erfindungsgemäßen Mittel in einer Menge von 0,01 bis 3 Gewichtsprozent, besonders bevorzugt in einer Menge von 0,1 bis 2,5 Gewichtsprozent, enthalten sind.

Die genannten Kuppler- und Entwicklersubstanzen können in dem Haarfärbemittel jeweils einzeln oder im Gemisch miteinander enthalten sein.

Die Gesamtmenge der in dem hier beschriebenen Haarfärbemittel enthaltenen Entwicklersubstanzen und Kupplersubstanzen beträgt etwa 0,1 bis 6,0 Gewichtsprozent, vorzugsweise 0,5 bis 4,0 Gewichtsprozent.

Weiterhin kann das erfindungsgemäße Haarfärbemittel dieser Anmeldung zusätzlich andere Farbkomponenten, beispielsweise mit sich selbst kuppelnde Farbvorstufen, wie zum Beispiel 6-Amino-2-methyl-phenol und 2-Amino-5-methyl-phenol, sowie ferner übliche direktziehende Farbstoffe, zum Beispiel Triphenylmethanfarbstoffe wie Diamond Fuchsine (C. I. 42 510) und Leather Ruby HF (C. I. 42 520), aromatische Nitrofarbstoffe wie 2-Nitro-1,4-diaminobenzol, 2-Amino-4-nitrophenol, 2-Amino-5-nitrophenol, 2-Amino-4,6-dinitrophenol, 2-Amino-5-(2′-hydroxyethylamino)-nitrobenzol und 4-(2′-Hydroxyethylamino)-3-nitrotoluol, 1-[(2′-Ureidoethyl)-amino]-4-nitrobenzol und Azofarbstoffe wie Acid Brown 4 (C. I. 14 805) und Dispersionsfarbstoffe, wie beispielsweise 1,4-Diaminoanthrachinon und 1,4,5,8-Tetraaminoanthrachinon, enthalten.

Weitere geeignete direkt auf das Haar aufziehende Farbstoffe sind beispielsweise im Buch von J.C. Johnson "Hair Dyes", Noyes Data Corp., Park Ridge, USA (1973), Seiten 3-91 und 113-139 (ISBV: 0-8155-0477-2) beschrieben.

Selbstverständlich können die Kuppler- und Entwicklersubstanzen sowie die anderen Farbkomponenten, sofern es Basen sind, auch in Form der physiologisch verträglichen Säureadditionssalze, wie beispielsweise als Hydrochlorid oder Sulfat oder ferner - falls sie aromatische OH-Gruppen besitzen - in Form der Salze mit Basen, zum Beispiel als Alkaliphenolate, eingesetzt werden.

Darüberhinaus können dem Haarfärbemittel noch weitere übliche kosmetische Zusätze, beispielsweise Antioxidantien wie Ascorbinsäure, Thioglykolsäure oder Natriumsulfit, sowie ferner Parfümöle, Komplexbildner, Netzmittel, Emulgatoren, Verdicker und Pflegestoffe, zugesetzt werden.

Die Zubereitungsform kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion. Die Zusammensetzung des neuen Haarfärbemittels stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beipielsweise Ethanol, Propanol und Isopropanol, oder Glykole wie 1,2-Propylenglykol, sowie Glycerin, weiterhin Netzmittel oder Emulgatoren aus den Klassen der anionischen, kationischen, amphoteren oder nichtionogenen oberflächenaktiven Substanzen, wie zum Beispiel Fettalkoholsulfate, oxethylierte Fettalkoholsulfate, Alkylsulfate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren, sowie außerdem Pflegestoffe wie kationische Harze, Lanolinderivate, Cholesterin,

Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin oder auch anorganische Basen wie Natriumhydroxid und Kaliumhydroxid, Verwendung finden.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form der 3 bis 12prozentigen, vorzugsweise 6prozentigen wäßrigen Lösungen, in Betracht. Wird eine 6prozentige Wasserstoffperoxidlösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und, falls notwendig, mit einer schwachen organischen Säure, wie zum Beispiel Zitronensäure oder Weinsaure, nachgespült. Anschließend wird das Haar getrocknet.

Die 4-Aminophenole der allgemeinen Formel (I) lassen sich alle gut, auch in größeren Mengen, nach dem nachfolgenden Reaktionsschema herstellen:

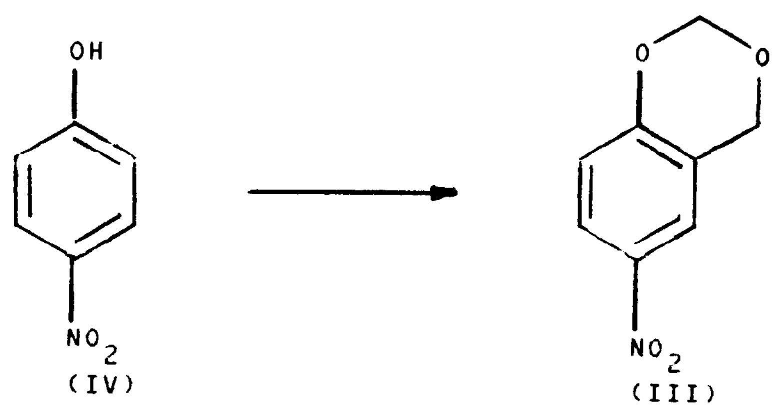

(IV) (III)

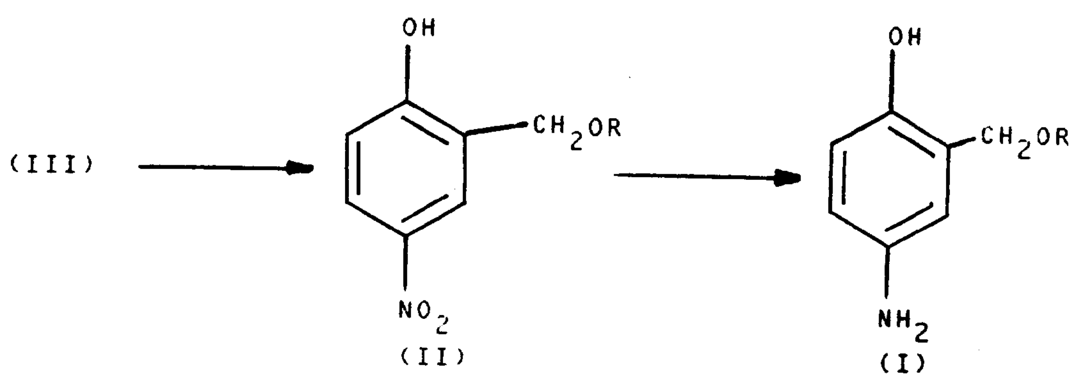

(II) (I)

Ausgehend von 4-Nitrophenol (IV), das technisch verfügbar ist, wird unter Einwirkung von Formalin und Schwefelsäure die Verbindung (III) erhalten (W. Borsche und A. D. Berghout, Ann. 330, 82 (1904), die sich unter

Ringöffnung mit den entsprechenden Alkoholen und Schwefelsäure zu den Benzylethern (II), wobei R die in der Formel (I) angegebene Bedeutung hat, umsetzen läßt (entsprechend I. Stavropvskaya, Z. Obsc. Chim. 24. 2068 (1954)). Die katalytische Hydrierung der Nitrogruppe liefert in guten Ausbeuten die 4-Aminophenol-derivate der allgemeinen Formel (I).

Die Herstellung einer Verbindung der Formel (I) wird im Beispiel 1 beschrieben. Die Herstellung der übrigen Verbindungen der Formel (I) erfolgt analog dazu.

Die Entwicklersubstanzen der Formel (I) sollen in den Haarfärbemitteln entweder als freie Basen oder in Form ihrer physiologisch verträglichen, wasserlöslichen Salze mit geeigneten anorganischen oder organischen Säuren, zum Beispiel als Chlorid, Sulfat, Phosphat, Acetat, Propionat, Lactat oder Citrat, eingesetzt werden. Die Verbindungen der Formel (I) sind gut in Wasser löslich, sie weisen außerdem eine ausgezeichnete Lagerstabilität, insbesondere als Bestandteil der hier beschriebenen Haarfärbemittel, auf.

Das erfindungsgemäße Haarfärbemittel auf der Basis der 4-Aminophenol-derivate der allgemeinen Formel (I) als Entwicklersubstanz ermöglicht Haarfärbungen mit ausgezeichneten Echtheitseigenschaften, insbesondere was die Licht-, Wasch- und Reibechtheit anbetrifft, welche sich mit Reduktionsmitteln wieder abziehen lassen.

Von besonderer Bedeutung ist weiterhin der durch die Verwendung dieser substituierten 4-Aminophenole in dem Haarfärbemittel gemäß vorliegender Anmeldung in toxikologischer und dermatologischer Hinsicht erzielte Fortschritt im Vergleich zu bekannten, für die Erzielung von Rottönen gebräuchlichen Entwicklersubstanzen, wie zum Beispiel 4-Aminophenol und 4-Amino-2-hydroxymethylphenol.

Hinsichtlich der färberischen Möglichkeiten bietet das erfindungsgemäße Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre gute Farbintensität wie auch Stabilität aus.

Dies wird insbesondere deutlich beim Vergleich von Haarfärbemitteln, die als Entwicklersubstanz einerseits die bekannten Entwicklersubstanzen p-Aminophenol, 4-Amino-2-methyl -phenol, 4-Amino-2-hydroxymethylphenol oder 4-Amino-3-methyl-phenol und andererseits die Entwicklersubstanzen der vorliegenden Anmeldung enthalten.

Während das 4-Amino-2-methyl-phenol und das 4-Amino-2-hydroxymethyl-phenol im Vergleich zum als Standard verwendeten p-Aminophenol schwächere und blaustichigere Färbungen liefern, führt die Verwendung der 4-Aminophenole der allgemeinen Formel (I) überraschenderweise zu etwa gleichen Farbtönen mit einer vergleichbaren Farbtiefe. Das 4-Amino-3-methylphenol, das ebenfalls eine strukturell ähnliche Verbindung darstellt, ergibt erheblich geringere Farbtiefen als die erfindungsgemäßen 4-Aminophenolderivate.

Die sehr guten färberischen Eigenschaften des Haarfärbemittels gemäß der vorliegenden Anmeldung zeigen sich weiterhin darin, daß dieses Mittel eine Anfärbung von ergrauten, chemisch nicht vorgeschädigten Haaren problemlos und mit guter Deckkraft ermöglicht.

Die nachstehenden Beispiele sollen den Gegenstand der Erfindung näher erläutern.

**Herstellungsbeispiel**

Beispiel 1: Herstellung von 4-Aminophenolen der allgemeinen Formel (I)

Stufe 1: 6-Nitro-1,3-benzodioxan (III):

10,8 g (0,08 Mol) 4-Nitrophenol werden unter Erwärmen in 12 ml 40 prozentigem Formalin gelöst und mit einem Gemisch aus 6 ml Wasser und 24 ml konzentrierter Schwefelsäure versetzt. Unter Rühren entsteht eine feste Masse, die nach 1 Stunde mit 200 ml Wasser versetzt und abgesaugt wird. Der Niederschlag wird aus Ethanol umkristallisiert. Nach dem Trocknen erhält man 8,5 g (60 Prozent der Theorie) leicht bräunliche Kristalle mit einem Schmelzpunkt von 146 bis 148 Grad Celsius.

Stufe 2: Darstellung der 2-Hydroxy-5-nitro-benzylether der Formel (II):

30 g (0,17 Mol) 6-Nitro-1,3-benzodioxan (III) werden in 300 ml des betreffenden Alkohols gelöst. Nach Zugabe von 15 ml Wasser werden unter Rühren 60 ml konzentrierte Schwefelsäure zugetropft. Die Lösung wird mit Hilfe der Kontrolle durch Dünnschichtchromatographie bis zur vollständigen Umsetzung der Ausgangsverbindung unter Rückfluß erhitzt. Nach dem Abkühlen wird das Reaktionsgemisch mit etwa 200 ml Wasser versetzt und die Lösung wird im Vakuum auf 1/3 ihres Volumens eingeengt, wobei sich ein gelbes öl abscheidet, das mit Ether extrahiert wird. Die Etherphase wird über Natriumsulfat getrocknet und der Rückstand mit Methylenchlorid als Laufmittel über Kieselgel filtriert. Nach dem Einengen der Methylenchloridlösung fallen leicht

gelbliche Kristalle der entsprechenden 4-Nitrophenolverbindung aus.

4-Nitrophenol-Derivate der allgemeinen Formel (II):

| R | Reaktionsdauer (Stunde) | Ausbeute (Prozent der Theorie) | Schmelzpunkt (Grad Celsius) |
|---|---|---|---|
| $-n-C_3H_7$ | 3 | 77 | 50 |
| iso-$C_3H_7$ | 2,5 | 55 | 67 |

Stufe 3: Reduktion der Nitrophenole der Formel (II):

Die Nitroverbindungen der allgemeinen Formel (II) werden in Methanol oder Ethanol unter Verwendung eines Palladium/Aktivkohle-Katalysators (5prozentig) mit Wasserstoff bei Raumtemperatur bis zur vollständigen Sättigung hydriert. Nach dem Abfiltrieren des Katalysators und dem Einengen der Lösung kristallisieren die 4-Aminophenole der allgemeinen Formel (I) aus, welche gegebenenfalls aus Aceton umkristallisiert werden. Durch Zugabe von methanolischer HCl-Lösung zum filtrierten Rohansatz und nachfolgendem Einengen der Lösung werden die Hydrochloride der erfindungsgemäßen 4-Aminophenolderivate erhalten.

Neue 4-Aminophenol-Derivate der allgemeinen Formel (I):

Ia: 4-Amino-2-propoxymethyl-phenol-hydrochlorid, graue Kristalle mit einem Schmelzpunkt von 260 Grad Celsius (unter Zersetzung)
$^1$H-NMR (DMSO-$d_6$) : δ = 0,91 (t, J = 7,4 Hz, 3 H, $-CH_2-CH_2-C\underline{H}_3$), 1,58 (m, 2 H, $-CH_2-C\underline{H}_2-CH_3$), 3,44 (t, J = 6,5 Hz, 2 H, $-C\underline{H}_2-CH_2-CH_3$), 4,42 (s, 2 H, $-C\underline{H}_2-O-$ ), 6,93 (d, J = 8,4 Hz, 1 H Aromaten-H), 7,13 (dd, J = 8,4 Hz und 2,2 Hz, 1 H, Aromaten-H), 7,28 (d, J = 2,2 Hz, 1 H, Aromaten-H), 10,17 (s, 3 H, $-O\underline{H}$ und $-N\underline{H}_2$, tauscht mit $D_2O$ aus).

Ib: 4-Amino-2-iso-propoxymethyl-phenol-hydrochlorid graue Kristalle mit einem Schmelzpunkt von 280 Grad Celsius (unter Zersetzung)
$^1$H-NMR (DMSO-$d_6$) : δ = 1,15 (d, J = 5 Hz, 6 H, $-CH(C\underline{H}_3)_2$, 3,67 (m, 1 H, $-C\underline{H}(CH_3)_2$, 4,43 (s, 2 H, $-C\underline{H}_2-O$), 6,93 (d, J = 8,5 Hz, 1 H, Aromaten-H), 7,13 (dd, J = 8,5 Hz und 2,6 Hz, 1 H, Aromaten-H), 7,28 (d, J = 2,6 Hz, 1 H, Aromaten-H), 10,01 (s, 1 H, $-O\underline{H}$, tauscht mit $D_2O$ aus), 10,19 (s, H, $-N\underline{H}_2$, tauscht mit $D_2O$ aus).

**Beispiel für Haarfärbemittel**

Beispiel 2: Haarfärbemittel in Cremeform

| | | |
|---|---|---|
| 1,30 | g | 4-Amino-2-iso-propoxymethyl-phenol-hydrochlorid |
| 1,10 | g | 1-Naphthol |
| 15,00 | g | Cetylalkohol |
| 0,30 | g | Natriumsulfit, wasserfrei |
| 3,50 | g | Laurylalkohol-diglykolethersulfat-Natriumsalz (28prozentige wäßrige Lösung) |
| 3,00 | g | Ammoniak, 22prozentige wäßrige Lösung |
| 75,80 | g | Wasser |
| 100,00 | g | |

50 g dieses Haarfärbemittels werden kurz vor dem Gebrauch mit 50 g Wasserstoffperoxidlösung (6prozentig) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine intensive Rotfärbung erhalten.

Alle in der vorliegenden Anmeldung angegebenen Prozentzahlen stellen, sofern nicht anders vermerkt, Gewichtsprozente dar.

**Patentansprüche**

1. Mittel zur oxidativen Färbung von Haaren mit einem Gehalt an mindestens einer Entwicklersubstanz und mindestens einer Kupplersubstanz, dadurch gekennzeichnet, daß es als Entwicklersubstanz ein 4-Aminophenol-derivat der allgemeinen Formel (I)

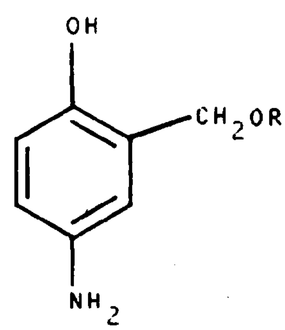

(I),

wobei R einen Propyl- oder iso-Propylrest, einen Aminoalkylrest mit 2 bis 4 Kohlenstoffatomen, einen Aminoalkylrest mit 2 bis 4 Kohlenstoffatomen, der an der Aminogruppe mit einem oder zwei Alkylresten, die 1 bis 4 Kohlenstoffatome aufweisen, substituiert ist, order einen Dihydroxyalkylrest mit 3 bis 4 Kohlenstoffatomen bedeutet, oder dessen physiologisch verträgliches, wasser -lösliches Salz enthält.

2. Mittel nach Anspruch 1, dadurch gekennzeichnet, daß das 4-Aminophenol-derivat der Formel (I) ausgewählt ist aus 4-Amino-2-propoxymethyl-phenol, 4-Amino-2-iso -propoxymethyl-phenol,

3. Mittel nach Anspruch 1 oder 2 dadurch gekennzeichnet, daß die Entwicklersubstanz der Formel I in einer Menge von 0,01 bis 3,0 Gewichtsprozent, vorzugsweise 0,1 bis 2,5 Gewichtsprozent, enthalten ist.

4. Mittel nach einem der Ansprüche 1 bis 3, dadurch gekennzeichnet, daß die Kupplersubstanz ausgewählt

ist aus 1-Naphthol, Resorcin, 4-Chlorresorcin, 4,6-Di-chlorresorcin, 2-Methylresorcin, 2-Amino-4-(2′-hydroxyethylamino)-anisol, 5-Amino-2-methylphenol, 2,4-Diaminophenoxyethanol, 4-Amino-2-hydroxy-phenoxyethanol, m-Aminophenol, 3-Amino-4-chlor-6-methylphenol, 3-Amino-2-methylphenol, 4-Hydroxy-1,2-methylendioxybenzol, 4-(2′-Hydroxyethylamino)-1,2-methylendioxybenzol, 2,4-Diamino-5-ethoxytoluol, 2,4-Diaminobenzylalkohol, m-Phenylendiamin, 4-Hydroxyindol, 3-Amino-5-hydroxy-2,6-dimethoxypyridin und 3,5-Diamino-2,6-dimethoxypyridin.

5. Mittel nach einem der Ansprüche 1 bis 4, dadurch gekennzeichnet, daß es die Kupplersubstanz in einer Menge von 0,01 bis 3.0 Gewichtsprozent, vorzugsweise 0,1 bis 2,5 Gewichtsprozent enthält.

6. Mittel nach einem der Ansprüche 1 bis 5, dadurch gekennzeichnet, daß die Gesamtmenge der Entwicklersubstanzen und Kupplersubstanzen 0,1 bis 6,0 Gewichtsprozent, vorzugsweise 0,5 bis 4,0 Gewichtsprozent beträgt.

7. Mittel nach einem der Ansprüche 1 bis 6, dadurch gekennzeichnet, daß es 6-Amino-2-methyl-phenol oder 2-Amino-5-methyl-phenol enthält.

8. Mittel nach einem der Ansprüche 1 bis 7, dadurch gekennzeichnet, daß es mindestens einen der direkt auf das Haar aufziehenden Farbstoffe Diamond Fuchsine (C.I. 42 510), Leather Ruby HF (C.I. 42 520), 2-Nitro-1,4-diaminobenzol, 2-Amino-4-nitrophenol, 2-Amino-5-nitrophenol, 2-Amino-4,6-dinitrophenol, 2-Amino-5-(2′-hydroxyethylamino)-nitrobenzol, 4-(2′-Hydroxyethylamino)-3-nitrotoluol, 1- (2′-Ureidoethyl)-amino -4-nitrobenzol, Acid Brown 4 (C.I. 14 805), 1,4-Diamino-anthrachinon und 1,4,5,8-Tetraaminoanthrachinon enthalt.

9. Mittel nach einem der Ansprüche 1 bis 8, dadurch gekennzeichnet, daß es einen pH-Wert von 8,0 bis 11,5 aufweist.

10. 4-Amino-2-propoxymethyl-phenol

11. 4-Amino-2-iso-propoxymethyl-phenol

## Claims

1. Agent for the oxidative colouring of hair containing at least one developer substance and at least one coupler susbstance, characterised in that it contains, as a developer substance, a 4-aminophenol derivative of the general formula (I)

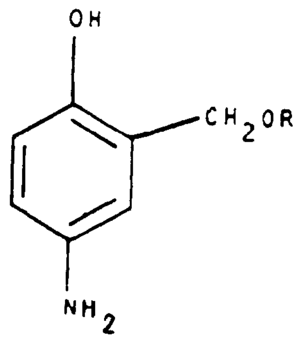

(I),

where R is a propyl or isopropyl residue, an aminoalkyl residue with 2 to 4 carbon atoms, an aminoalkyl residue with 2 to 4 carbon which is substituted at the amino group with one or two alkyl having comprising 1 to 4 carbon atoms or a dihydroxyalkyl residue with 3 to 4 carbon atoms, or its physiologically compatible, water-soluble salt.

2. Agent according to claim 1, characterised in that the 4-aminophenol derivative of formula (I) is selected from 4-amino -2-propoxymethylphenol, 4-amino-2-iso-propoxymethylphenol.

3. Agent according to claim 1 or 2, characterised in that the developer substance of formula I is present in

an amount of between 0.01 and 3.0 per cent by weight, preferably 0.1 and 2.5 per cent by weight.

4. Agent according to any one of claims 1 to 3, characterised in that the coupler substance is selected from: 1-naphthol, resorcinol, 4-chlororesorcinol, 4,6-dichlororesorcinol, 2-methylresorcinol, 2-amino-4-(2′-hydroxyethylamino)-anisol, 5-amino-2-methylphenol, 2,4-diaminophenoxyethanol, 4-amino-2-hydroxyphenoxyethanol, m-aminophenol, 3-amino-4-chloro-6-methylphenol, 3-amino-2-methylphenol, 4-hydroxy-1,2-methylenedioxybenzene, 4-(2′-hydroxyethylamino)-1,2-methylenedioxybenzene, 2,4-diamino-5-ethoxytoluene, 2,4-diaminobenzylalcohol, m-phenylenediamine, 4-hydroxindole, 3-amino-5-hydroxy-2,6-dimethoxypyridine and 3.5-diamino-2,6-dimethoxypyridine.

5. Agent according to any one of claims 1 to 4, characterised in that it contains the coupler substance in an amount of from 0.01 to 3.0 per cent by weight, preferably 0.1 to 2.5 per cent by weight.

6. Agent acccording to one of claims 1 to 5, characterised in that the total amount of developer substances and coupler substances is from 0.1 to 6.0 per cent by weight, preferably 0.5 to 4.0 per cent by weight.

7. Agent according to one of claims 1 to 6, characterised in that it contains 6-amino-2-methyl-phenol or 2-amino-5-methylphenol.

8. Agent according to one of claims 1 to 7, characterised in that it contains at least one of the colorants, which is applied directly to the hair, Diamond Fuchsine (C.i. 42 510), Leather Ruby HF (C.i. 42 520), 2-nitro-1,4-diaminobenzene, 2-amino-4-nitrophenol, 2-amino-5-nitrophenol, 2-amino-4,6-dinitrophenol, 2-amino-5-(2′-hydroxyethylamino)-nitrobenzene, 4-(2′-hydroxyethylamino)-3-nitrotoluene, 1-(2′-ureidoethyl)-amino-4-nitrobenzene, Acid Brown 4 (C.i. 14 805), 1,4-diamino-anthraquinone and 1,4,5,8-tetra-aminoanthraquinone.

9. Agent according to one of claims 1 to 8, characterised in that is has a pH of from 8.0 to 11.5.

10. 4-amino-2-propoxymethylphenol.

11. 4-amino-2-iso-propoxymethylphenol.

## Revendications

1. Produit pour la teinture des cheveux par oxydation contenant au moins un développeur et au moins un copulateur, caractérisé en ce qu'il contient comme développeur un dérivé du 4-aminophénol de la formule générale

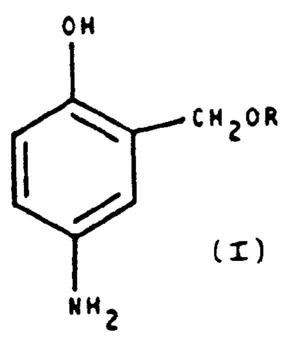

(I) ,

dans laquelle R est un radical propyle ou iso-propyle, un radical aminoalcoyle ayant 2 à 4 atomes de carbone, substitué sur le groupe amino par un ou deux radicaux alcoyle ayant 1 à 4 atomes de carbone, ou un radical dihydroxyalcoyle ayant 3 à 4 atomes de carbone soluble dans l'eau et physiologiquement compatible.

2. Produit selon la revendication 1, caractérisé en ce qu'on choisit le dérivé d'un 4-aminophénol de la formule (I) dans la série des 4-amino-2-propoxyméthyl-phénol, 4-amino-2-iso-propoxyméthyl-phénol.

3. Produit selon la revendication 1 ou la revendication 2, caractérisé en ce que le développeur de la formule I est contenu en une quantité comprise entre 0,01 et 3,0 % en poids, de préférence entre 0,1 et 2,5 % en poids.

4. Produit selon l'une des revendications 1 à 3, caractérisé en ce que le copulateur est choisi dans la série des 1-naphtol, résorcine, 4-chlororésorcine, 4,6-dichlororésorcine, 2-méthyl-résorcine, 2-amino-4-(2'-hydroxyéthylamine)-anisol, 5-amino-2-méthylphénol, 2,4-diaminophénoxyéthanol, 4-amino-2-hydroxyphénoxyéthanol, m-aminophénol, 3-amino-4-chloro-6-méthylphénol, 3-amino-2-méthylphénol, 4-hydroxy-1,2-méthylènedioxybenzéne, 4-(2'-hydroxyéthylamino)-1,2-méthylènedioxy -benzène, 2,4-diamino-5-éthoxytoluène, 2,4-diaminobenzylalcool, m-phénylènediamine, 4-hydroxyindol, 3-amino-5-hydroxy-2,6-diméthoxy-pyridine et 3,5-diamino-2,6-diméthoxypyridine.

5. Produit selon l'une des revendications 1 à 4, caractérisé en ce qu'il contient le copulateur en une quantité comprise entre 0,01 et 3,0 % en poids, de préférence entre 0,1 et 2,5 % en poids.

6. Produit selon l'une des revendications 1 à 5, caractérisé en ce que la quantité totale du développeur et du copulateur est comprise entre 0,1 et 6,0 % en poids, de préférence entre 0,5 et 4,0 % en poids.

7. Produit selon l'une des revendications 1 à 6, caractérisé en ce qu'il contient du 6-amino-2-méthylphénol ou du 2-amino-5-méthylphénol.

8. Produit selon l'une des revendications 1 à 7, caractérisé en ce qu'il contient au moins un des colorants prenant directement sur le cheveu, à savoir Diamond Fuchsine (I. C. 42 510), Leather Ruby HF (I. C. 42 520), 2-nitro-1,4-diaminobenzène, 2-amino-4-nitrophénol, 2-amino-5-nitrophénol, 2-amino-4,6-dinitrophénol, 2-amino-5-(2'hydroxy-éthylamino)-nitrobenzène, 4-(2'-hydroxyéthylamino)-3-nitrotoluène, 1-(2-uréidoéthyl)-amino-4-nitrobenzène, Acid Brown 4 (I. C. 14 805), 1,4-diamino-anthraquinone et 1,4,5,8-tétraaminoanthraquinone.

9. Produit selon l'une des revendications 1 à 8, caractérisé en ce qu'il a un pH compris entre 8,0 et 11,5.

10. 4-amino-2-propoxyméthyl-phénol.

11. 4-amino-2-iso-propoxyméthyl-phénol.